(19) Europäisches Patentamt
European Patent Office
Office européen des brevets

(11) Numéro de publication: **0 252 823**
**B1**

(12) # FASCICULE DE BREVET EUROPEEN

(45) Date de publication du fascicule du brevet: **11.07.90**

(21) Numéro de dépôt: **87401552.2**

(22) Date de dépôt: **02.07.87**

(51) Int. Cl.$^5$: **C 07 D 513/04,**
C 07 D 519/00,
A 61 K 31/435, A 61 K 31/505
// (C07D513/04, 277:00, 209:00),(C07D519/00, 513:00, 471:00)

(54) **Nouveaux dérivés du 1H, 3H-pyrrolo 1,2-cr thiazole, leur préparation et les compositions pharmaceutiques qui les contiennent.**

(30) Priorité: **04.07.86 FR 8609729**

(43) Date de publication de la demande:
**13.01.88 Bulletin 88/02**

(45) Mention de la délivrance du brevet:
**11.07.90 Bulletin 90/28**

(84) Etats contractants désignés:
**AT BE CH DE ES FR GB GR IT LI LU NL SE**

(56) Documents cités:
**EP-A-0 115 979**

(73) Titulaire: **RHONE-POULENC SANTE**
**20, avenue Raymond Aron**
**F-92160 Antony (FR)**

(72) Inventeur: **Fabre, Jean-Louis**
**9, rue Fagon**
**F-75013 Paris (FR)**
Inventeur: **James, Claude**
**31 bis, avenue Gambetta**
**F-75020 Paris (FR)**
Inventeur: **Lavé, Daniel**
**72 boulevard de la Villette**
**F-75019 Paris (FR)**

(74) Mandataire: **Pilard, Jacques et al**
**RHONE-POULENC SANTE, Service Brevets, 20 Avenue Raymond Aron**
**F-92165 Antony Cédex (FR)**

**Le dossier contient des informations techniques présentées postérieurement au dépôt de la demande et ne figurant pas dans le présent fascicule.**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 252 823 B1

**Description**

La présente invention concerne de nouveaux dérivés du 1H,3H-pyrrolo [1,2-c] thiazole de formule générale:

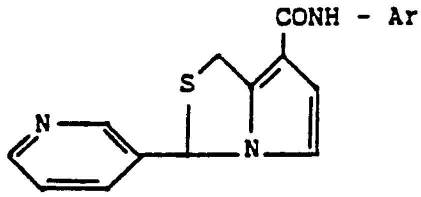

(I)

dans laquelle Ar représente un radical pyridyle, substitué par un ou plusieurs atomes d'halogène ou radicaux alcoyle, alcoyloxy, alcoylthio, trifluorométhyle, amino, alcoylamino, dialcoylamino, hydroxy, cyano, alcoylsulfinyle, alcoylsulfonyle, sulfamido, alcoylsulfamido, dialcoylsulfamido, alcoylcarbonyle, benzoyle, alcoyloxycarbonyle, carboxy, phénoxycarbonyle, alcoylcarbonyloxy, benzoyloxy, alcoylcarbonylamino, benzamido, phényle, benzyle, phénoxy ou phénylthio étant entendu que i) les radicaux alcoyle et portions alcoyle contiennent 1 à 4 atomes de carbone en chaîne doite ou ramifiée, ii) les radicaux phényle ou portions phényle peuvent être non substitués ou substitués par un ou plusieurs atomes d'halogène ou radicaux alcoyle, alcoyloxy, alcoylthio, trifluorométhyle, amino, alcoylamino, dialcoylamino, hydroxy, cyano, phényle, ou benzyle et que iii) l'invention concerne les produits racémiques, les énantiomères et leurs mélanges, ainsi que leurs sels acceptables.

Selon l'invention, les produits de formule générale (I) peuvent être préparés par action d'une amine de formule générale:

$$ArNH_2 \qquad (II)$$

dans laquelle Ar est défini comme précédemment sur un acide de formule:

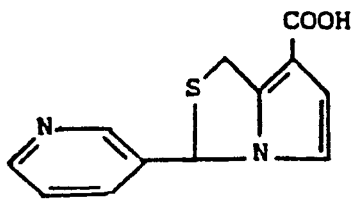

(III)

ou un dérivé réactif de cet acide.

Il est en effet particulièrement avantageux d'utiliser l'acide de formule (III) sous une forme activée telle que le chlorure d'acide ou de le faire réagir avec le N,N'-carbonyl-diimidazole ou un chloroformiate d'alcoyle avant de condenser l'amine de formule générale (II).

Généralement il est préférable d'employer le chlorure d'acide et d'effectuer la réaction dans un solvant organique tel que le chloroforme, le chlorure de méthylène ou le dioxanne à une température comprise entre 0°C et la température de reflux du mélange réactionnel, en présence d'un accepteur d'acide tel que la triéthylamine.

L'acide de formule (III) racémique peut être préparé selon la méthode décrite dans le brevet européen n° 0 115 979.

Les amines de fomule générale (II) peuvent être préparées par application ou adaptation des méthodes déjà décrites dans la littérature.

La présence en position 3 du cycle pyrrolo [1,2-c] thiazole d'un carbone asymétrique fait que les produits de formule générale (I) selon l'invention peuvent exister sous forme de racémiques ou sous forme d'énantiomères. Généralement le procédé décrit précédement conduit aux produits racémiques mais il est entendu que l'on peut obtenir directement les énantiomères correspondants si l'on met en oeuvre le procédé en opérant avec un acide de formule (III) optiquement actif.

L'acide de fomule (III) optiquement actif peut être préparé selon l'un ou l'autre des procédé suivants:

A — Premier procédé: On saponifie un ester optiquement actif de pouvoir rotatoire correspondant de formule générale:

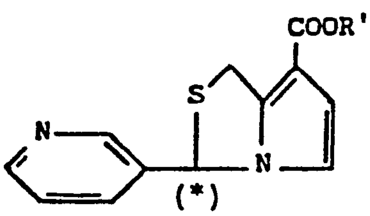

(IV)

dans laquelle R' représente un radical alcoyle contenant 1 à 4 atomes de carbone en chaîne droite ou ramifiée et le symbole * représente le signe + ou le signe — selon que le produit mis en oeuvre est dextrogyre ou lèvogyre.

Généralement la saponification s'effectue par toute méthode douce connue de l'homme de métier pour transformer un ester en acide sans racémiser les centres chiraux présents dans la molécule. Il est particulièrement avantageux d'effectuer la saponification au moyen d'un hydroxyde de métal alcalin tel que la soude ou la potasse à une température comprise entre 20 et 50°C.

L'ester de formule générale (IV) peut être obtenu en faisant réagir le produit de réaction du chlorure de p-toluène sulfonyle, de la triéthylamine et de l'acide de formule:

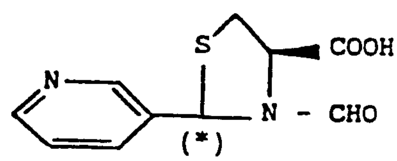

(V)

dans laquell le symbole * a la même signification que dans l'ester (IV) correspondant défini précédemment sur le produit de réaction de la triéthylamine et d'un dichloro-2,3 propionate d'alcoyle de formule générale:

$ClCH_2$—CH—COOR'
|
Cl

(VI)

dans laquelle R' est défini comme précédemment au sein d'un solvant organique tel que le dichloro-1,2 éthane ou le chlorure de méthylène à une température comprise entre 20°C et la température de reflux du mélange réactionnel.

L'acide de formule générale (V) peut être obtenu par action d'un mélange d'acide formique et d'anhydride acétique sur un acide de formule:

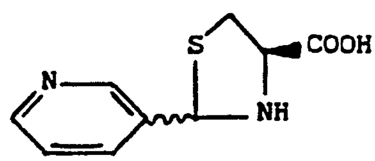

(VII)

et séparation ultérieure des formes dextrogyre et l'évogyre en opérant selon les méthodes habituelles, par exemple par recristallisation et/ou formation de sels avec des bases optiquement actives comme l'α-méthylbenzylamine, séparation de ces sels et libération de l'acide correspondant.

B — Deuxième procédé: On sépare les énantiomères de l'acide de formule (III) par toute méthode connue de l'home du métier, notamment par formation d'un sel avec une base optiquement active telle que les formes optiquement actives de l'α-méthylbenzylamine, recristallisations du sel obtenu et décomposition de celui-ci par un acide tel que l'acide chlorhydrique.

Selon l'invention, les produits de formule générale (I) dextrogyres, lévogyres ou racémiques, peuvent également être préparés en faisant réagir le produit de réaction du chlorure de p-toluènesulfonyle, de la triéthylamine et de l'acide dextrogyre, lévogyre ou racémique correspondant de formule (V) sur le produit de réaction de la triéthylamine et d'un produit de formule générale:

$ClCH_2$—CH—CONHAr
|
Cl

(VIII)

dans laquelle Ar est défini comme précédemment.

On opère généralement au sein d'un solvant organique tel que le dichloro-1,2 éthane ou le chlorure de méthylène à une température comprise entre 20°C et la température de reflux du mélange réactionnel.

Les produits de formule générale (VIII) peuvent être préparés par application ou adaptation de méthodes connues dans la littérature, notamment par action du chlorure de dichloro-2,3 propionyle sur une amine de formule générale (II) définie comme précédemment, en opérant dans le toluène à une température comprise entre 20°C et la température de reflux du mélange réactionnel.

Il est entendu pour l'homme du métier que, pour la mise en oeuvre du procédé décrit précédemment, il peut être nécessaire d'introduire des groupements protecteurs au niveau de certaines fonctions présentes dans le radical Ar des différents produits mis en oeuvre. Le groupement protecteur pourra être ensuite

éliminé au moment le plus propice de la synthèse. Ainsi, lorsque le radical Ar est porteur d'une fonction amino ou alcoylamino, celle-ci pourra être protégée par exemple par un radical tert-butyloxycarbonyle puis libérée après réaction au moyen d'un acide aqueux, par exemple au moyen d'une solution aqueuse d'acide chlorhydrique, ou mieux au moyen d'une solution acétique de gaz chlorhydrique. Lorsque le radical Ar est porteur d'une fonction hydroxy, celle-ci pourra être avantageusement protégée sous forme de radical tétrahydropyranyloxy ou méthoxyméthyloxy, puis libérée après réaction par hydrolyse. Lorsque le radical Ar est porteur d'une fonction carboxylique, celle-ci pourra être avantageusement protégée sous forme d'ester d'alcoyle qui pourra être saponifié pour donner l'acide correspondant selon les méthodes habituelles.

Les nouveaux produits de formule générale (I) peuvent être transformés en sel d'addition avec les acides par action d'un acide dans un solvant organique tel qu'un alcool, une cétone, un éther ou un solvant chloré. Le sel précipite, généralement après concentration de sa solution; il est séparé par filtration ou décantation.

Les nouveaux produits de formule générale (I) contenant dans leur molécule un groupement acide peuvent être transformés en sels métalliques ou en sels d'addition avec les bases azotées, par toute méthode connue de l'homme du métier pour effectuer celle salification sans toucher au reste de la molécule.

Lorsque, dans la présente description, on se réfère à un produit indentifié par son nom chimique, il est entendu qu'en l'absence de spécification particulière concernant la nature des isomères, on se réfère toujours au produit racémique correspondant.

Les nouveaux produits selon l'invention ainsi que leurs sels d'addition présentent des propriétés phamacologiques intéressants associées à une faible toxicité. Ils se sont montrés actifs à des concentrations inhibitrices ($CI_{50}$) comprises entre 1 et 1000 nM dans le test d'antagonisme de la fixation du [$^3$H] O-octadécyl-1-O-acétyl-2 ns-glycérophosphorylcholine-3 (P.A.F.-acéther tritié) sur ses sites récepteurs des plaquettes sanguines selon la technique suivante:

a) Préparation des plaquettes lavées de lapin

Des lapins mâles néozélandais (hybrid HY 2000) d'un poids de 2,5 kg environ sont ponctionnés à l'artère de l'oreille. Le sang est recueilli sur un mélange d'acide citrique (1,9 mM), de citrate trisodique (9 mM), de phosphate monosodique (1,75 mM) et de dextrose (5,6 mM). Le sang est centrifugé à 150 g pendant 20 minutes à 15°C. On obtient ainsi le plasma riche en plaquettes (PRP). Ce plasma est centrifugé à 1000 g pendant 15 minutes à 15°C. Le culot plaquettaire ainsi obtenu est lavé une première fois par une solution de Tyrode modifiée contenant 0,35% de sérum albumine bovine (BSA), 2 mMole $MgCl_2$, 0,2 mMole d'EGTA, puis par une solution de Tyrode sans EGTA. Les plaquettes sont finalement mises en suspension dans un tampon d'essai (tampon A) ayant la composition suivante: NaCl (140 mM), KCl (2,7 mM), $NaH_2PO_4$ (0,4 mM), $MgCl_2$ (2 mM), $NaHCO_3$ (12 mM), tampon Tris, HCl (10 mM), dextrose (6,2 mM) et BSA (0,25%). La concentration finale de la suspension est ajustée à $4.10^8$ plaquettes/$cm^3$ au moyen de ce tampon.

b) Mise en oeuvre du test proprement dit.

Dans un tube de 5 $cm^3$, on introduit successivement le tamon d'essai A décrit precédemment, le produit à étudier, le PAF-acéther tritié (0,5 nMole — activité spécifique : 80 Ci/mMole) et les plaquettes obtenues comme décrit précédemment ($0,5.10^8$ plaquettes) de manière à obtenir un volume final de 0,5 $cm^3$ et on laisse le mélange incuber pendant une heure à 20°C. On ajoute alors 2 $cm^3$ de tampon A refoidi à 4°C, filtre rapidement le contenu du tube sur filtre WHATMAN CF/C (marque déposée) et rince le tube très rapidement 3 fois avec 2 $cm^3$ de tampon A refroidi à 4°C. Le filtre est séché et placé dans une fiole contenant 4,5 $cm^3$ de liquide scintillant READY SOLV. MP (N. D. BECKMAN) et la radioactivité est mesurée au moyen d'un compteur universel RACK BETA 1218 LKB. On détermine ainsi la radioactivité liée totale.

La fixation spécifique du PAF-acéther tritié est déterminée en retranchant de la radioactivité liée totale, la radiocativité restée sur le filtre après addition de 10 µMole de N-(méthoxy-3-phényl) (pyridyl-3)-3 1H,3H-pyrrolo [1,2-c] thiazolecarboxamide-7. Pour chaque produit à étudier, on reproduit l'essai 3 fois a des concentrations croissantes allant de $10^{-10}$ à $10^{-4}$ M. On détermine graphiquement la $CI_{50}$ pour chaque produit par analyse en log Probit de la courbe d'inhibition.

On sait que le PAF-acéther intervient dans un grand nombre de maladies et de troubles tels que les réactions allergiques (asthme ou bronchite) ou les réactions inflammatoires des muqueuses gastriques et intestinales d'origines deiverses et notamment les réactions inflammatoires dues aux irradiations et aux chocs endotoxiniques ainsi que les troubles liés à l'agrégation des plaquettes. Le PAF-acéther libéré au cours de ces désordres se fixe sur des récepteurs spécifiques de ce médiateur. Le test de liaison aux récepteurs des plaquettes sanguines décrit précédemment est l'un des modèles expérimentaux possibles pour étudier l'aptitude des produits à se fixer à ces récepteurs.

Les produits selon l'invention déplacent le PAF-acéther de ses sites de liaison. Ils entrent donc en compétition avec lui et en antagonisent les effets. Ainsi il est prévisible que les produits selon l'invention auront un rôle thérapeutique dans le traitement des maladies et des états énumérés ci-dessus.

On connait déjà par le brevet européen 0 115 979 des pyrrolothiazoles qui ont une certaine action inhibitrice vis-à-vis du PAF-acéther mais les produits selon la présente invention se fixent aux récepteurs des plaquettes à des doses plus faibles et sont donc plus aptes à inhiber les effets du PAF-acéther.

Par ailleurs, les produits selon l'invention présentent une toxicité faible. Leur $DL_{50}$ par voie orale est

généralement comprise entre 300 et 900 mg/kg chez la souris par voie orale.

Sont particulièrement intéressants les produits de formule générale (I) dans laquelle Ar repésente un radical pyridyle, substitué par un ou plusieurs atomes d'halogène ou radicaux alcoyle ou alcoyloxy étant entendu que i) les radicaux alcoyle et portions alcoyle contiennent 1 à 4 atomes de carbone en chaîne droite ou ramifiée, et que ii) les produits concernés sont les produits racémiques, les énantiomères et leurs mélanges.

Sont plus particulièrement intéressants les produits de formule générale (I) dans laquelle Ar représente un radical pyridyle substitué par un atome d'halogène ou un radical alcoyle.

D'un intérêt tout particulier sont les produits suivants:

(+)N-(méthyl-4 pyridyl-2) (pyridyl-3)-3 1H,3H-pyrrolo [1,2-c] thiazolecarboxamide-7

N-(méthyl-4 pyridyl-2) (pyridyl-3)-3 1H,3H-pyrrolo [1,2-c] thiazolecarboxamide-7

N-(chloro-6 pyridyl-2) (pyridyl-3)-3 1H,3H-pyrrolo [1,2-c] thiazolecarboxamide-7

N-(chloro-4 pyridyl-2) (pyridyl-3)-3 1H,3H-pyrrolo [1,2-c] thiazolecarboxamide-7.

D'une façon générale, les produits selon l'invention qui sont les plus intéressants sont ceux qui se présentent sous la forme racémique et les isomères optiques de forme dextrogyre.

Pour l'emploi thérapeutique, il peut être fait usage des nouveaux produits de formule générale (I), tels quels ou, les cas échéant, à l'état de sels pharmaceutiquement acceptables, c'est à-dire non toxiques aux doses d'utilisation.

Come sels pharmaceutiquement acceptables peuvent être cités les sels d'addition avec les acides minéraux tels que chlorhydrates, sulfates, nitrates, phosphates ou organiques tels que acétates, propionates, succinates, bezoates, fumarates, maléates, méthanesulfonates, iséthionates, théophillineacétates, salicylates, phénolphtalinates, méthylène-bis-β-oxynaphtoates ou des dérivés de subsitution de ces composés.

Lorsqu'ils peuvent exister, on peut encore citer les sels avec les métaux alcalins tels que les sels de sodium, potassium ou lithium, avec les métaux alcalino-terreux tels que les sels de calcium ou de magnésium, et les sels d'addition avec les bases organiques tels que les sels d'éthanolamine ou de lysine.

Les exemples suivants, donnés à titre non limitatif, montrent comment l'invention peut être mise en pratique.

Exemple 1

A une solution de 3,9 g d'amino-2 chloro-6 pyridine et de 6,1 g de triéthylamine dans 200 $cm^3$ de dioxanne chauffée à une température voisine de 60°C, on ajoute en 5 minutes à une température comprise entre 60 et 68°C, 9 g de chlorhydrate de chloroformyl-7- (pyridyl-3)-3 1H, 3H-pyrrolo [1,2-c] thiazole. La suspension obtenue est chauffée sous agitation à une température voisine de 100°C pendant 5 heures et 45 minutes puis est agitée à une température voisine de 20°C pendant 16 heures. Le solvant est évaporé sous pression réduite (20 mm de mercure; 2,7 kPa) à une température voisine de 60°C. La résidu est dissous dans 500 $cm^3$ de chlorure de méthylène. La solution obtenue est lavée 2 fois par 300 $cm^3$ au total d'eau distillée, 2 fois par 300 $cm^3$ au total d'une solution aqueuse de soude 2N et 2 fois par 300 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 9,5 g de produit brut que l'on dissout dans 140 $cm^3$ de butanol-1 bouillant. La solution obtenue est additionnée de 0,5 g de noir décolorant et filtrée à chaud. Le filtrat est refroidi à une température voisine de 4°C pendant 16 heures. Les cristaux apparus sont séparés par filtration, lavés 3 fois par 30 $cm^3$ au total de butanol-1 et 3 fois par 150 $cm^3$ au total d'éther diéthylique puis séchés sous pression réduite (20 mm de mercure; 2,7 kPa) à une température voisine de 20°C en présence de potasse en pastilles. On obtient ainsi 5,6 g de N-(chloro-6 pyridyl-2) (pyridyl-3-)-3 1H,3H-pyrrolo [1,2-c] thiazolecarboxamide-7 sous forme de cristaux crème fondant à 186°C.

L'amino-2 chloro-6- pyridine peut être préparée selon J. P. WIBAUT et J. R. NICOLAI, Rec. Trav. Chim., *58*, 709 (1939).

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 2

A une solution de 4,5 g d'amino-2 fluoro-6 pyridine et de 8,1 g de triéthylamine dans 250 $cm^3$ de dioxanne chauffée à une température voisine de 60°C, on ajoute, en 5 minutes, à une température comprise entre 60 et 70°C, 12 g de chlorhydrate de chloroformyl-7 (pyridyl-3)-3 1H,3H pyrrolo [1,2-c] thiazole. La suspension obtenue est chauffée à une température voisine de 100°C pendant 6 heures et 45 minutes puis est agitée à une température voisine de 20°C pendant 16 heures. Le solvant est évaporé sous pression réduite (20 mm de mercure; 2,7 kPa) à une température voisine de 60°C. Le résidue obtenu est dissous dans 500 $cm^3$ de chlorure de méthylène et la solution obtenue est lavée 2 fois par 300 $cm^3$ au total d'eau distillée, 2 fois par 300 $cm^3$ au total d'une solution aqueuse de soude 2N et 2 fois par 300 $cm^3$ au total d'eau distillée puis séchée sur de sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 11,4 g de produit brut que l'on dissout dans 70 $cm^3$ de butanol-1 bouillant. La solution obtenue est additionnée de 0,5 g de noir decolorant et filtrée à chaud. Le filtrat est refroidi à une température voisine

de 4°C pendant 2 heures. Les cristaux apparus sont séparés par filtration, lavés 3 fois par 45 $cm^3$ au total de butanol-1, 2 fois par 50 $cm^3$ au total d'éthanol et 3 fois par 90 $cm^3$ au total d'éther diéthylique puis séchés sous pression réduite (20 mm de mercure; 2,7 kPa) à une température voisine de 20°C en présence de potasse en pastilles. On obtient ainsi 5,3 g de N-(fluoro-6 pyridyl-2) (pyridyl-3)-3 1H,3H-pyrrolo [1,2-c] thiazolecarboxamide-7 sous forme de cristaux beiges fondant à 198°C.

L'amino-2 fluoro-6 pyridine peut être préparée selon la méthode décrite dans le brevet britannique n° 2 078 738.

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 3

A une solution de 2,2 g d'amino-2 picoline-6 et de 4,05 g de triéthylamine dans 130 $cm^3$ de dioxanne chauffée à une température voisine de 60°C, on ajoute, en 5 minutes, à une température comprise entre 60 et 66°C, 6 g de chlorhydrate de chloroformyl-7 (pyridyl-3)-3 1H,3H, pyrrolo [1,2-c] thiazole. La suspension obtenue est chauffée sous agitation à une température voisine de 100°C pendant 7 heures et 30 minutes puis est agitée à une température voisine de 20°C pendant 16 heures. Le solvant est évaporé sous pression réduite (20 mm de mercure; 2,7 kPa) à une température voisine de 60°C. Le résidu est dissous dans 300 $cm^3$ de chlorure de méthylène et la solution obtenue est lavée 2 fois par 300 $cm^3$ au total d'eau distillée, 2 fois par 300 $cm^3$ au total d'une solution aqueuse de soude 2N et 2 fois par 300 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 5,6 g de produit brut que l'on dissout dans 60 $cm^3$ d'acétonitrile bouillant. La solution obtenue est additionnée de 0,5 g de noir décolorant et filtrée à chaud. Le filtrat est refroidi à une température voisine de 4°C pendant 2 heures. Les cristaux apparus sont séparés par filtration, lavés 2 fois par 20 $cm^3$ au total d'acétonitrile refroidi à une température voisine de 4°C puis 2 fois par 50 $cm^3$ au total d'éther diéthylique et séchés sous pression réduite (20 mm de mercure; 2,7 kPa) à une température voisine de 20°C en présence de potasse en pastilles. On obtient ainsi 2,5 g de N-(méthyl-6 pyridyl-2) (pyridyl-3)-3 1H,3H-pyrrolo [1,2-c] thiazolecarboxamide-7 sous forme de cristaux beige clair fondant à 162°C.

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 4

A une solution de 2,5 g d'amino-2 méthoxy-6 pyridine et de 4,1 g de triéthylamine dans 80 $cm^3$ de dioxanne chauffée à une température voisine de 40°C, on ajoute, en 15 minutes, à une température voisine de 40°C, 6 g de chlorhydrate de chloroformyl-7 (pyridyl-3)-3 1H,3H, pyrrolo [1,2-c] thiazole. La suspension obtenue est chauffée sous agitation à une température voisine de 100°C pendant 6 heures puis est agitée à une température voisine de 20°C pendant 12 heures. Le solvant est évaporé sous pression réduite (20 mm de mercure; 2,7 kPa) à une température voisine de 60°C. Le résidu est dissous dans 300 $cm^3$ de chlorure de méthylène et la solution obtenue est lavée 2 fois par 200 $cm^3$ au total d'eau distillée, 2 fois par 200 $cm^3$ au total d'une solution aqueuse de soude 2N et 5 fois par 500 $cm^3$ au total d'eau distillée puis séchée sur de sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 5,6 g de produit que l'on chromatographie sur une colonne de 6 cm de diamètre conténant 480 g de silice (0,04—0,063 mm). On élue par un mélange d'acétate d'éthyle et de méthanol (95—5 en volumes) sous une pression de 0,4 bar (41 kPa) en recueillant des fractions de 75 $cm^3$. Les 9 premières sont éliminées. Les 6 fractions suivantes sont réunies et concentrées à sec sous pression réduite (20 mm de mercure; 2,7 kPa) à une température voisine de 50°C. On obtient ainsi 3,6 g der produit brut fondant à 151°C. Ce produit est dissous dans 50 $cm^3$ d'éthanol bouillant. La solution obtenue est additionnée de 0,5 g de noir décolorant et filtrée à chaud. Le filtrat obtenu est refroidi à une température voisine de 4°C pendant 2 heures. Les cristaux apparus sont séparés par filtration, lavés 2 fois par 10 $cm^3$ au total d'éthanol refroidi à une température voisine de 4°C et 2 fois par 20 $cm^3$ au total d'ether diéthylique puis séchés sous pression réduite (20 mm de mercure; 2,7 kPa) à une température voisine de 20°C en présence de potasse en pastilles. On obtient ainsi 3 g de N-(méthoxy-6 pyridyl-2) (pyridyl-3)-3 1H,3H-pyrrolo [1,2-c] thiazolecarboxamide-7 sous forme de cristaux blancs fondant à 154°C.

L'amino-2 méthoxy-6 pyridine peut être préparée selon A. F. BICKEL et J. P. WIBAUT, Rec. Trav. Chim., *65*, 65 (1946).

Le chlorhydrate de chloroformyl-7 (pyridyl-3)-3 1H,3H-pyrolo [1,2-c] thiazole est préparé selon la méthode décrite dans le brevet européen n° 0 115 979.

Exemple 5

A une solution de 2,6 g d'amino-2 chloro-4 pyridine et de 4,05 g de triéthylamine dans 150 $cm^3$ de dioxanne chauffée à une température voisine de 67°C, on ajoute, en 15 minutes, à une température voisine de 67°C, 6 g de chlorhydrate de chloroformyl-7 (pyridyl-3)-3-1H,3H, pyrrolo [1,2-c] thiazole. La suspension obtenue est chauffée sous agitation à une température voisine de 100°C pendant 7 heures et 30 minutes puis est agitée à une température voisine de 20°C pendant 16 heures. Le solvant est évaporé sous pression

réduite (20 mm de mercure; 2,7 kPa) à une température voisine de 60°C. Le résidue est dissous dans 350 $cm^3$ de chlorure de méthylène. La solution obtenue est lavée 2 fois par 200 $cm^3$ au total d'eau distillée, 2 fois par 200 $cm^3$ au total d'une solution aqueuse de soude N et 3 fois par 300 $cm^3$ au total d'eau distillée puis séchée sur de sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 5 g de produit brut que l'on dissout dans 200 $cm^3$ d'acétonitrile bouillant. La solution obtenue est additionnée de 0,5 g de noir décolorant et filtrée à chaud. Le filtrat est refroidi à une température voisine de 4°C pendant 2 heures. Les cristaux apparus sont séparés par filtration, lavés 2 fois par 10 $cm^3$ au total d'acétonitrile refroidi à une température voisine de 4°C et 2 fois par 20 $cm^3$ au total d'ether diéthylique puis séchés sous pression réduite (20 mm der mercure; 2,7 kPa) à une température voisine de 20°C en présence de potasse en pastilles. On obtient ainsi 2,6 g de N-(chloro-4 pyridyl-2) (pyridyl-3)-3 1H,3H-pyrrolo [1,2-c] thiazolecarboxamide-7 sous forme de cristaux crème fondant à 190°C.

L'amino-2 chloro-4 pyridine peut être obtenue selon R. GRAF, Chem. Ber., *64*, 21 (1931).

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 6

A une solution de 2,45 g d'amino-2 éthyl-6 pyridine et de 4,05 g de triéthylamine dans 150 $cm^3$ de dioxanne chauffée à une température voisine de 68°C, on ajoute, en 5 minutes, à une température comprise entre 68 et 75°C, 6 g de chlorhydrate de chloroformyl-7 (pyridyl-3)-3 1H, 3H, pyrrolo [1,2-c] thiazole. La suspension obtenue est chauffée sous agitation à une température voisine de 100°C pendant 6 heures et 15 minutes puis est agitée à une température de 20°C pendant 16 heures. Le solvant est évaporé sous pression réduite (20 mm de mercure; 2,7 kPa) à une température voisine de 60°C. Le résidu est dissous dans 300 $cm^3$ de chlorure de méthylène. La solution obtenue est lavée 2 fois par 300 $cm^3$ au total d'eau distillée, 2 fois par 300 $cm^3$ au total d'une solution aqueuse de soude 2N et 2 fois par 300 $cm^3$ au total d'eau distillée puis séchée sur de sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 6,5 g de produit brut que l'on dissout dans 75 $cm^3$ d'acétonitrile bouillant. La solution obtenue est additionnée de 0,5 g de noir décolorant et filtrée à chaud. Le filtrat est refroidi à une température voisine de 4°C pendant 16 heures. Les cristaux apparus sont séparés par filtration, lavés 2 fois par 10 $cm^3$ au total d'acétonitrile refroidi à une température voisine de 4°C et 3 fois par 90 $cm^3$ au total d'ether diéthylique puis séchés sous pression réduite (20 mm de mercure; 2,7 kPa) à une température voisine de 20°C en présence de potasse en pastilles. On obtient ainsi 2,3 g de N-(éthyl-6 pyridyl-2) (pyridyl-3)-3 1H,3H-pyrrolo [1,2-c] thiazolecarboxamide-7 sous forme de cristaux crème fondant à 150°C.

L'amino-2 éthyl-6 pyridine peut être préparée selon S. J. CHILDRESS et J. V. SCUDI, J. Org. Chem., *23*, 67 (1958).

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 7

A une solution de 2,1 g d'amino-2 méthyl-4 pyridine et de 4,05 g de triéthylamine dans 100 $cm^3$ de dioxanne chauffée à une température voisine de 65°C, on ajoute, en 20 minutes à une température comprise entre 65 et 72°C, 6 g de chlorhydrate de chloroformyl-7 (pyridyl-3)-3 1H,3H, pyrrolo [1,2-c] thiazole. La suspension obtenue est chauffée sous agitation à une température voisine de 100°C pendant 5 heures et 45 minutes puis est agitée à une température voisine de 20°C pendant 16 heures. Le solvant est évaporé sous pression réduite (20 mm de mercure; 2,7 kPa) à une température voisine de 60°C. Le résidue est dissous dans 250 $cm^3$ de chlorure de méthylène. La solution obtenue est lavée par 100 $cm^3$ d'eau distillée, puis par 100 $cm^3$ d'une solution aqueuse de soude 4N et 3 fois par 450 $cm^3$ au total d'eau distillée puis séchée sur de sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 4,8 g de produit brut que l'on dissout dans 50 $cm^3$ d'acétonitrile bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 10 $cm^3$ au total d'acétonitrile refroidi à une température voisine de 4°C, puis séchés sous pression réduite (20 mm der mercure; 2,7 kPa) à une température voisine de 20°C en présence de potasse en pastilles. On obtient ainsi 1,5 g de N-(méthyl-4 pyridyl-2) (pyridyl-3)-3 1H,3H-pyrrolo [1,2-c] thiazolecarboxamide-7 sous forme de cristaux crème fondant à 163°C.

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 8

A une solution de 4,3 g d'amino-2 méthyl-4 pyridine et de 8,1 g de triéthylamine dans 300 $cm^3$ de dioxanne chauffée à une température voisine de 60°C, on ajoute, en 5 minutes à une température comprise entre 65 et 72°C, 12 g de chlorhydrate du chlorure d'acide provenant de l'acide (+) (pyridyl-3)-3 1H,3H, pyrrolo [1,2-c] thiazole carboxylique-7. La suspension obtenue est chauffée sous agitation à une

température voisine de 100°C pendant 7 heures puis est agitée à une température voisine de 20°C pendant 16 heures. Le solvant est évaporé sous pression réduite (20 mm de mercure; 2,7 kPa) à une température voisine de 60°C. Le résidu est dissous dans 500 $cm^3$ de chlorure de méthylène. La solution obtenue est lavée 2 fois par 300 $cm^3$ au total d'eau distillée, 2 fois par 300 $cm^3$ au total d'une solution aqueuse saturée de bicarbonate de sodium et 2 fois par 300 $cm^3$ au total d'eau distillée puis séchée sur de sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 13,3 g de produit brut que l'on dissout dans 140 $cm^3$ d'acétonitrile bouillant. La solution obtenue est additionnée de 0,5 g de noir décolorant et filtrée à chaud. Le filtrat est refroidi à une température voisine de 20°C pendant 2 heures. Les cristaux apparus sont séparés par filtration, lavés 3 fois par 75 $cm^3$ au total d'acétonitrile et 3 fois par 90 $cm^3$ au total d'éther diéthylique puis séchés sous pression réduite (20 mm de mercure; 2,7 kPa) à une température voisine de 20°C en présence de potasse en pastilles. On obtient ainsi 3,1 g de (+) N(méthyl-4 pyridyl-2) (pyridyl-3)-3 1H,3H-pyrrolo [1,2-c] thiazolecarboxamide-7 sous forme de cristaux crème fondant à 174°C.
($[\alpha]_D^{20}$ = +82° ± 0,9°; c = 1,09 diméthylformamide).

Le chlorhydrate du chlorure d'acide provenant de l'acide (+) (pyridyl-3)-3 1H,3H-pyrrolo [1,2-c] thiazolecarboxylique-7 est préparé de la façon suivante: Une suspenson de 20,9 g d'acide (+) (pyridyl-3)-3 1H,3H-pyrrolo [1,2-c] thiazolecarboxylique-7 dans un mélange de 52,1 g der chlorure de thionyle, de 0,1 $cm^3$ de diméthylformamide et de 290 $cm^3$ de dichloro-1,2 éthane est chauffée à une température voisine de 80°C pendant 3 heures. Après refoidissement à une température voisine de 20°C, les cristaux sont séparés par filtration, lavés 3 fois par 150 $cm^3$ au total de dichloro-1,2 éthane, 3 fois par 150 $cm^3$ d'éther diéthylique puis séché sous pression réduite (20 mm de mercure; 2,7 kPa) à une température voisine de 20°C. On obtient ainsi 24,5 g de chlorhydrate du chlorure d'acide provenant de l'acide (+) (pyridyl-3)-3 1H,3H-pyrrolo [1,2-c] thiazolecarboxylique-7 sous forme de cristaux crème à 175°C.

L'acide (+) (pyridyl-3)-3 1H,3H-pyrrolo [1,2-c] thiazolecarboxylique-7 peut être obtenu de la façon suivante selon l'une ou l'autre des procédés suivants:

A — Premier procédé : On chauffe à une température voisine de 40°C pendant 14 heures une solution de 19,5 g de (+) (pyridyl-3)-3 1H,3H-pyrrolo [1,2-c] thiazolecarboxylate-7 d'ethyle et de 11,9 g de potasse en pastilles dans un mélange de 70 $cm^3$ d'éthanol et de 70 $cm^3$ d'eau distillée. Le solvant est évaporé sous pression réduite (20 mm de mercure; 2,7 kPa) à une température voisine de 40°C. Le résidu est dissous dans 200 $cm^3$ d'eau distillée et la solution obtenue est amenée à un pH voisin de 4 par addition de 250 $cm^3$ d'une solution aqueuse d'acide chlorhydrique N et est agitée à une température voisine de 20°C pendant 1 heure. Les cristaux apparus sont séparés par filtration, lavés 5 fois par 250 $cm^3$ au total d'eau distillee, 5 fois par 150 $cm^3$ au total d'éthanol et 3 fois par 90 $cm^3$ au total d'éther diéthylique puis séchés sous pression réduite (20 mm de mercure; 2,7 kPa) à une température voisine de 20°C en présence de potasse en pastilles. On obtient 14,1 g de produit brut fondant à 210°C. Ce produit est dissous dans 420 $cm^3$ d'ethanol bouillant; la solution obtenue est additionnée de 0,5 g de noir décolorant et filtrée à chaud. Le filtrat est refroidi à une température voisine de 4°C pendant 16 heures. Les cristaux apparus sont séparés par filtration, lavés 3 fois par 90 $cm^3$ au total d'ethanol et 3 fois par 150 $cm^3$ au total d'éther diéthylique puis séchés sous pression réduite (20 mm de mercure; 2,7 kPa) à une température voisine de 20°C. On obtient ainsi 10,3 g d'acide (+) (pyridyl-3)-3 1H,3H-pyrrolo [1,2-c] thiazolecarboxylique-7 sous forme de cristaux crème fondant à 210°C. ($[\alpha]_D^{20}$ = +163° ± 1,6° (c = 1,08; soude N).

Le (+)(pyridyl-3)-3 1H,3H-pyrrolo [1,2-c] thiazolecarboxylate-7 d'éthyle peut être préparé de la façon suivante : A une suspension de 23,8 g d'acide N-formyl (pyridyl-3)-2 thiazolidinecarboxylique-4-(2R,4R) dans 90 $cm^3$ de dichloro-1,2 éthane, on ajoute en 2 minutes, à une température comprise entre 20 et 27°C, 11,2 g de triéthylamine. La suspension obtenue est agitée à une température voisine de 20°C pendant 1 heure et la solution obtenue est ajoutée en 50 minutes à une température voisine de 20°C, à une solution de 21 g de chlorure de paratoluènesulfonyle dans 110 $cm^3$ de dichloro-1,2 éthane. On obtient une solution trouble (solution A). Par ailleurs, à une solution de 18,6 g dichloro-2,3 propionate d'éthyle dans 100 $cm^3$ de dichloro-1,2 éthane, on ajoute en 15 minutes, à une température comprise entre 20 et 30°C, 33,4 g de triéthylamine. A la suspension (suspension B) obtenue et agitée à une température voisine de 20°C pendant 50 minutes, on ajoute en 50 minutes à une température comprise entre 20 et 36°C la solution A préparée précédemment. La suspension obtenue est agitée pendant 1 heure et 40 minutes à une température voisine de 40°C puis pendant 20 minutes à une température voisine de 60°C. Après refroidissement à une température voisine de 20°C, la suspension obtenue est additionnée de 100 $cm^3$ d'au distillée. La phase organique est séparée, lavée 3 fois par 300 $cm^3$ au total d'eau distillée, 2 fois par 300 $cm^3$ au total d'une solution aqueuse saturée de bicarbonate de sodium puis 2 fois par 300 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient 25,6 g de produit brut que l'on dissout dans 250 $cm^3$ d'acétate d'éthyle. La solution obtenue est extraite 3 fois par 300 $cm^3$ au total d'une solution aqueuse d'acide chlorhydrique 2N. Les extraits aqueux sont réunis, lavés par 250 $cm^3$ d'acétate d'éthyle et amenés à un pH voisin de 8 par addition de bicarbonate de sodium. La suspension obtenue est extraite une première fois par un mélange de 250 $cm^3$ d'éther diéthylique et de 250 $cm^3$ d'acetate d'éthyle puis 3 fois par 450 $cm^3$ au total d'acétate d'éthyle. Les extraits organiques sont réunis, lavés 2 fois par 300 $cm^3$ au total d'eau distillée, séchés sur du sulfate de magnésium anhydre,

additionnés de 0,5 g de noir decolorant filtrés, additionnés de 30 g de silice (0,020—0,045 mm), filtrés et concentrés à sec sous pression réduite (20 mm de mercure; 2,7 kPa) à une température voisine de 60°C. On obtient ainsi 19,6 g de (+) (pyridyl-3)-3 1H,3H-pyrrolo [1,2-c] thiazolecarboxylate-7 d'ethyle sous forme d'une huile orangée. Rf = 0,5 (chromatographie sur couche mince de gel de silice éluant : acétate d'ethyle); $[\alpha]_D^{20} = +115° \pm 1°$ (c = 1,51 diméthylformamide).

Le dichloro-2,3 propionate d'éthyle peut être préparé d'aprés la méthode décrite dans le brevet japonais n° 81 87531 [Chem. Abstr., *95*, 203335, (1981)].

L'acide N-formyl (pyridyl-3)-2 thiazolidinecarboxylique-4-(2R,4R) peut être obtenue de la façon suivante : A 420 $cm^3$ d'acide formique, on ajoute en 25 minutes à une température voisine de 10°C, 340 g d'anhydride acétique. La solution obtenue est agitée à une temperature voisine de 10°C pendant 30 minutes puis est additionnée en 50 minutes à une température voisine de 10°C de 233 g d'acide (pyridyl-3)-2 thiazolidinecarboxylique-4-(2RS,4R). La solution obtenue est agitée à une température voisine de 10°C pendant 30 minutes puis à une température voisine de 20°C pendant 16 heures. Le solvant est évaporé sous pression réduite (20 mm de mercure; 2,7 kPa) à une température voisine de 60°C. Le résidu obtenue est mis en suspension dans 2600 $cm^3$ d'ethanol bouillant. La suspension obtenue est refroidie à une température voisine de 4°C pendant 2 heures. Les cristaux apparus sont séparés par filtration, lavés 2 fois par 530 $cm^3$ au total d'éthanol refroidi à une température voisine de 4°C et séchés à l'aire. On obtient ainsi 245 g de produit fondant à 230°C. 60 g de ce produit sont dissous dans 540 $cm^3$ d'éthanol aqueux à 50% à l'ébullition. La solution obtenue est refroidie à une température voisine de 10°C pendant 2 heures. Les cristaux apparus sont separes par filtration, lavés 3 fois par 300 $cm^3$ au total d'éthanol et 3 fois par 450 $cm^3$ au total d'éther diéthylique puis séchés sous pression réduite (20 mm de mercure; 2,7 kPa) à une température voisine de 20°C en présence de potasse en pastilles. On obtient ainsi 48,2 g d'acide N-formyl (pyridyl-3)-2 thiazolidinecarboxylique-4-(2R, 4R) sous forme de cristaux blancs fondant à 250°C.
$[\alpha]_D^{20} = + 100° \pm 1°$ (c = 1,37; soude N).

L'aide (pyridyl-3)-2 thiazolidinecarboxylique-4(2RS,4R) peut être préparé selon A. BANASHEK et M. I. SHCHUKINA, J. Gen. Chem. U.S.S.R., *31*, 1374 (1961); Chem. Abstr. *55*, 24739h, (1961).

B — Deuxième procédé : 200 g d'acide (±) (pyridyl-3)-3 1H,3H-pyrrolo[1,2-c] thiazolecarboxylique-7 et 147 g de L (−)α-méthylbenzylamine sont dissous dans 1000 $cm^3$ d'isopropanol bouillant. La solution obtenue est additionnée de 0,5 g de noir décolorant et filtrée à chaud. Le filtrat est refroidi à une température voisine de 20°C pendant 16 heures. Les cristaux apparus sont séparés par filtration, lavés 3 fois par 450 $cm^3$ au total d'isopropanol refroidi à une température voisine de 4°C et 3 fois par 600 $cm^3$ au total d'éther diéthylique puis séchés sous pression réduite (20 mm de mercure; 2,7 kPa) à une température voisine de 20°C en présence de potasse en pastilles. On obtient 134,6 g de produit que l'on dissout dans 500 $cm^3$ d'isopropanol bouillant. La solution obtenue est additionnée de 0,5 g de noir décolorant et filtrée à chaud. Le filtrat est refroidi à une température voisine de 20°C pendant 16 heures. Les cristaux apparus sont séparés par filtration, lavés 3 fois par 300 $cm^3$ au total d'isopropanol refroidi à une température voisine de 4°C et 2 fois par 400 $cm^3$ d'éther diéthylique puis séchés sous pression réduite (20 mm de mercure; 2,7 kPa) à une température voisine de 20°C en présence de potasse en pastilles. On obtient 88,3 g de produit que l'on dissout dans 500 $cm^3$ d'isopropanol bouillant; la solution obtenue est filtrée à chaud et le filtrat est refroidi à une température voisine de 4°C pendant 16 heures. Les cristaux apparus sont séparés par filtration, lavés 2 fois par 100 $cm^3$ au total d'isopropanol refroidi à une température voisine de 4°C et 3 fois par 300 $cm^3$ au total d'éther diethylique puis séchés sous pression réduite (20 mm de mercure; 2,7 kPa) à une température voisine de 20°C en presence de potasse en pastilles. On obtient 77,3 g de sel de L(−)α-méthylbenzylamine de l'acide (+) (pyridyl-3)-3 1H,3H-pyrrolo [1,2-c] thiazolecarboxylique-7 sous forme de cristaux crème fondant à 154°C.
$[\alpha]_D^{20} = +110° \pm 2°$ (c = 1,01; eau).

Ce produit est dissous dans 600 $cm^3$ d'eau distillée à une température voisine de 65°C. La solution obtenue est filtrée à chaud et refroidie à une température voisine de 10°C et est amenée à un pH voisin de 3,5 par addition d'acide chlorhydrique concentré à une température comprise entre 10 et 15°C. Les cristaux apparus sont séparés par filtration, lavés 3 fois par 600 $cm^3$ au total d'eau distillée, 2 fois par 160 $cm^3$ au total d'éthanol et 2 fois par 200 $cm^3$ au total d'éther diéthylique puis séchés sous pression réduite (20 mm de mercure; 2,7 kPa) à une température voisine de 20°C en présence de potasse en pastilles. On obtient 48 g de produit que l'on dissout dans 1000 $cm^3$ d'éthanol bouillant; la solution obtenue est additionnée de 0,5 g de noir décolorant et filtrée à chaud. Le filtrat obtenue est refroidi à une température voisine de 4°C pendant 2 heures. Les cristaux apparus sont séparés par filtration, lavés 2 fois par 60 $cm^3$ au total d'éthanol refroidi à une température voisine de 4°C puis 2 fois par 200 $cm^3$ d'éther diethylique puis séchés sous pression réduite (20 mm de mercure; 2,7 kPa) à une température voisine de 20°C en presence de potasse en pastilles. On obtient ainsi 42,5 g d'acide (+) (pyridyl-3)-3 1H,3H-pyrrolo [1,2-c] thiazolidinecarboxylique-7 sous forme de cristaux jaunes fondant à 210°C.
$[\alpha]_D^{20} = + 168° \pm 2°$ (c = 1,02; NaOH N).

L'acide (±) (pyridyl-3)-3 1H,3H-pyrrolo [1,2-c] thiazolecarboxylique-7 peut être préparé selon la méthode décrite dans le brevet européen n° 0 115 979.

Exemple 9

A une solution de 2,4 g d'amino-2 diméthyl-4,6 pyridine et de 4,1 g de triéthylamine dans 100 $cm^3$ de

dioxanne chauffée à une température voisine de 60°C, on ajoute, en 25 minutes à une température comprise entre 60 et 70°C, 6 g de chlorhydrate de chloroformyl-7 (pyridyl-3)-3 1H, 3H, pyrrolo [1,2-c] thiazole. La suspension obtenue est chauffée sous agitation à une température voisine de 100°C pendant 5 heures et 30 minutes puis est agitée à une température voisine de 20°C pendant 16 heures. Le solvant est évaporé sous pression réduite (20 mm de mercure; 2,7 kPa) à une température voisine de 50°C. Le résidu est dissous dans 400 $cm^3$ de chlorure de méthylène. La solution obtenue est lavée par 100 $cm^3$ d'eau distillée, 2 fois par 200 $cm^3$ au total d'une solution aqueuse de soude N et 3 fois par 300 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 6,3 g de produit brut que l'on dissout dans 140 $cm^3$ d'acétonitrile bouillant. La solution obtenue est additionnée de 0,5 g de noir décolorant et filtrée à chaud. Le filtrat est refroidi à une température voisine de 20°C pendant 1 heure. Les cristaux apparus sont séparés par filtration, lavés 2 fois par 20 $cm^3$ au total d'acétonitrile refroidi à une température voisine de 4°C puis séchés sous pression réduite (20 mm de mercure; 2,7 kPa) à une température voisine de 20°C en présence de potasse en pastilles. On obtient ainsi 3,9 g de N-(diméthyl-4,6 pyridyl-2) (pyridyl-3)-3 1H,3H-pyrrolo [1,2-c] thiazolecarboxamide-7 sous forme de cristaux blancs fondant à 171°C.

Le chlorhydrate du chloroformyl-7 (pyridyl-3)-3 1H,3H-pyrrolo [1,2-c] thiazole est préparé selon la méthode décrite dans le brevet eurpéen n° 115 979.

La présente invention concerne également les médicaments contsitués par un produit de formule générale (I), sous forme, libre ou sous forme de sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon linvention peuvent être utilisés par voie orale, parentérale, rectale ou topique.

Comme compositions solides pour administration orale peuvent être utilisés des comprimés, pilules, poudres (notamment dans des capsules de gelatine ou des cachets) ou granulés. Dans ces compositions, le produit actif selon l'invention est mélangé à un ou plusieures diluants inertes tels que amidon, cellulose, saccharose, lactose ou silca. Ces compositions peuvent également comprendre des substances autres que les diluants, par exemple un ou plusieurs lubrifants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptable contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épassissants, aromatisants ou stabilisants.

Les compositions stériles pour administration parentérale peuvent être de préférence des solutions aqueuses ou non aqueuses, des suspensions ou des émulsions. Comme solvant ou vèhicule, on peut employer l'eau, le propylénglycol, un polyéthylèneglycol, des huiles végétales, en particulier l'huile d'olive, des esters organiques injectables, par exemple l'oléate d'ethyle ou autres solvants organiques convenables.

Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacoa, des glycérides semi-synthétiques ou des polyéthylènglycols.

Les compositions pour administration topique peuvent être par exemple des crèmes, pommades, lotions, collyres, collutoires, gouttes nasales ou aérosols.

En thérapeutique humain, les produits selon l'invention sont particulièrement utiles dans le traitement de toutes les conditions pathologiques dans lesquelles le PAF-acéther peut ête incriminé, directement ou indirectement, notamment les affections allergiques, inflammatoires et du système digestif tels que les ulcères, les colites et les lésions intestinales causées par les irradiations ou les chocs endotoxiniques. Les doses dépendent de l'effet recherché et de la durée du traitment; elles sont généralement comprises entre 25 et 300 mg par jour par voie orale, intraveineuse ou par inhalation pour un adulte en une ou plusieurs prises.

D'une façon générale, le médicin déterminera la posologie qu'il estime la plus appropriée en fonction de l'âge, du poids et de tous les autres facteurs propres au subjet à traiter.

Les exemples suivants, donnés, à titre non limitatif, illustrent des compositions selon l'invention.

Exemple A

On prépare, selon la technique habituelle, des comprimés dosés à 25 mg de produit actif ayant la composition suivante:

| | |
|---|---|
| N-(chloro-6 pyridyl-2) (pyridyl-3)-3 1H, 3H-pyrolo [1,2-c] thiazolecarboxamide-7 | 25 mg |
| amidon | 60 mg |
| lactose | 50 mg |
| stéarate de magnésium | 2 mg |

Exemple B

On prépare, selon la technique habituelle, une solution injectable contenant 5 mg de produit actif ayant la composition suivante:

| | |
|---|---|
| (+)N-(méthyl-4 pyridyl-2) (Pyridyl-3)-3 1H,3H-pyrrolo [1,2-c] thiazolecarboxamide-7 | 5 mg |
| solution d'acide chlorhydrique 0,01N | 0,29 $cm^3$ |
| soluté injectable | 2 $cm^3$ |

**Revendications pour les Etats contractants: BE CH DE FR GB IT LI LU NL SE**

1. Nouveau dérivé du 1H,3H-pyrrolo [1,2-c] thiazole, caractérisé en ce qu'il répond à la formule générale:

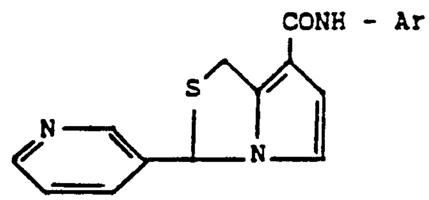

(I)

dans laquelle Ar représente un radical pyridyle, substitué par un ou plusieurs atomes d'halogène ou radicaux alcoyle, alcoyloxy, alcoythio, trifluorométhyle, amino, alcoylamino, dialcoylamino, hydroxy, cyano, alcoylsulfinyle, alcoylsulfonyle, sulfamido, alcoylsulfamido, dialcoylsulfamido, alcoylcarbonyle, benzoyle, alcoyloxycarbonyle, carboxy, phénoxycarbonyl, alcoylcarbonyloxy, benzoyloxy, alcoylcarbonylamino, benzamido, phényle, benzyle, phénoxy ou phénylthio étant entendu que i) les radicaux alcoyle et portions alcoyle contiennent 1 à 4 atomes de carbone en chaîne droite ou ramifiée, ii) les radicaux phényle ou portions phényle peuvent être non substitués ou substitués par un ou plusieurs atomes d'halogène ou radicaux alcoyle, alcoyloxy, alcoylthio, trifluorométhyle, amino, alcoylamino, dialcoylamino, hydroxy, cyano, phényle, ou benzyle et que iii) l'invention concerne les produits racémiques, les énantiomères et leurs mélanges, ainse que les sels acceptables.

2. Procédé de préparation de nouveaux dérivés selon la revendication 1, caractérisé en ce que l'on fait agir une amine de formule générale:

$$ArNH_2 \qquad (II)$$

dans laquelle Ar est défini comme à la revendication 1, sur un acide racémique ou optiquement actif de formule:

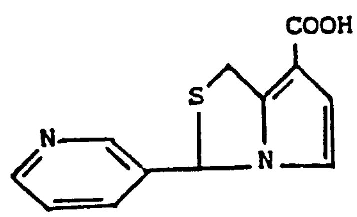

(III)

ou un dérivé réactif de cet acide, puis isole le produit obtenu et la transforme éventuellement en un sel pharmaceutiquement compatible.

3. Procédé de préparation de nouveaux dérivés selon la revendication 1, caractérisé en ce que l'on fait réagir le produit de réaction du chlorure de p-toluènesulfonyle, e la triéthylamine et de l'acide de formule:

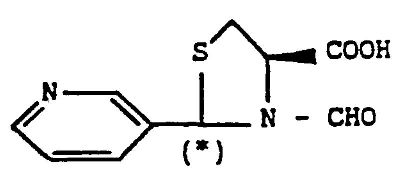

(V)

dans laquelle le symbole * représente le signe +, le signe − ou le signe ± qu'on met en oeuvre un produit dextrogyre, lévogyre ou racémique sur le produit de réaction de la triéthylamine et d'une produit de formule générale:

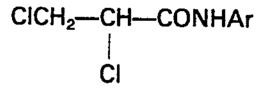

(VIII)

dans laquelle Ar est défini comme à la revendication 1, puis isole le produit obtenu et le transforme éventuellement en un sel pharmaceutiquement acceptable.

4. Composition pharmaceutique, caractérisée en ce qu'elle contient comme produit actif au moins un dérivé selon la revendication 1, en association avec un ou plusieure diluants ou adjuvants compatibles et pharmaceutiquement acceptables.

**Revendications pour l'Etat contractant: AT ES GR**

Procédé de préparation d'un nouveau dérivé du 1H, 3H-pyrrolo [1,2-c] thiazole, de formule générale:

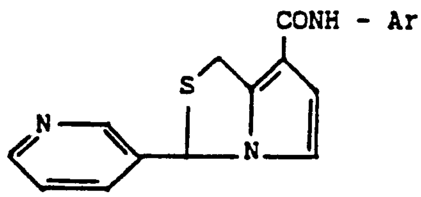

(I)

dans laquelle Ar représente un radical pyridyle, substitué par un ou plusieurs atomes d'halogène ou radicaux alcoyle, alcoyloxy, alcoythio, trifluorométhyle, amino, alcoylamino, dialcoylamino, hydroxy, cyano, alcoylsulfinyle, alcoylsulfonyle, sulfamido, alcoylsulfamido, dialcoylsulfamido, alcoylcarbonyle, benzoyle, alcoyloxycarbonyle, carboxy, phénoxycarbonyl, alcoylcarbonyloxy, benzoyloxy, alcoylcarbonylamino, benzamido, phényle, benzyle, phénoxy ou phénylthio étant entendu que i) les radicaux alcoyle et portions alcoyle contiennent 1 à 4 atomes de carbone en chaîne droite ou ramifiée, ii) les radicaux phényle ou portions phényle peuvent être non substitués ou substitués par un ou plusieurs atomes d'halogène ou radicaux alcoyle, alcoyloxy, alcoylthio, trifluorométhyle, amino, alcoylamino, dialcoylamino, hydroxy, cyano, phényle ou benzyle et que iii) l'invention concerne les produits racémiques, les énantiomères et leurs mélanges, ainse que les sels acceptables caractérisé en ce que:

A — on fait agir une amine de formule générale:

$$ArNH_2 \qquad (II)$$

dans laquelle Ar est défini comme précédemment, sur un acide racémique ou optiquement actif de formule:

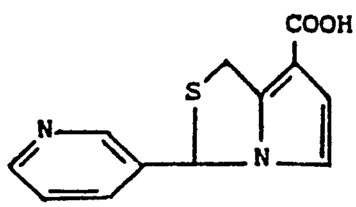

(III)

ou un dérivé réactif de cet acide, puis isole le produit obtenue et le transforme éventuellement en un sel d'addition pharmaceutiquement compatible ou en ce que

B — on fait réagir le produit de réaction du chlorure de p-toluènesulfonyle, de la triéthylamine et de l'acide de formule:

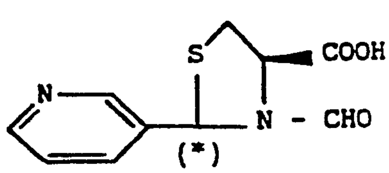

(V)

dans laquelle le symbole * représente le signe +, le signe − ou le signe ± qu'on met en oeuvre un produit dextrogyre, lévogyre ou racémique sur le produit de réaction de la triéthylamine et d'un produit de formule générale:

$$ClCH_2-\underset{\underset{\displaystyle Cl}{|}}{CH}-CONHAr \qquad (VIII)$$

dans laquelle Ar est défini comme précédemment, puis isole le produit obtenu et le transforme éventuellement en un sel pharmaceutiquement acceptable.

**Patentansprüche für die Vertragsstaaten: BE CH DE FR GB IT LI LU NL SE**

1. Neues Derivat des 1H, 3H-Pyrrolo[1,2-c]thiazol, dadurch gekennzeichnet, daß es der allgemeinen Formel I

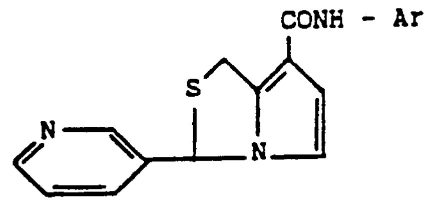

(I)

entspricht, worin Ar einen Pyridylrest bedeutet, substituiert durch ein oder mehrere Halogenatome oder Reste Alkyl, Alkyloxy, Alklthio, Trifluormethyl, Amino, Alkylamino, Dialkylamino, Hydroxy, Cyano, Alkylsulfinyl, Alkylsulonyl, Sulfamido, Alkylsulfamido, Dialkylsulfamido, Alkylcarbonyl, Benzoyl, Alkyloxycarbonyl, Carboxy, Phenoxycarbonyl, Akyl-carbonyloxy, Benzoyloxy, Alkyl-carbonylamino, Benzamido, Phenyl, Benzyl, Phenoxy oder Phenylthio, wobei i) die Alkylreste und Alkylteile 1 bis 4 Kohlenstoffatome in gerader oder verzweigter kette enthalten, ii) die Phenylreste oder Phenylteile nicht substituiert oder substituiert durch eine oder mehrere Halogenatome oder Alkyl-, Alkyloxy-, Alkylthio-, Trifluormethyl-, Amino-, Alkylamino-, Dialkylamino-, Hydroxy-, Cyano, Phenyl- oder Benzylreste sein können und iii) die Erfindung die racemischen Produkte, die Enantiomeren und ihre Gemische, sowie die pharmazeutisch annehmbaren Salze betrifft.

2. Verfahren zur Herstellung der neuen Derivate gemäß Anspruch 1, dadurch gekennzeichnet, daß man ein Amin der allgemeinen Formel

$$Ar-NH_2 \qquad (II)$$

worin Ar wie in Anspruch 1 definiert ist, auf eine racemische oder optisch aktive Säure der Formel

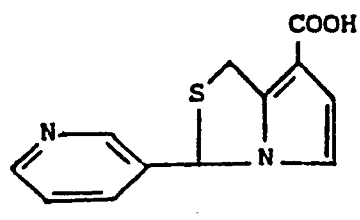

(III)

oder ein reaktives Derivat dieser Säure einwirken läßt, dann das erhaltene Produkt isoliert und es gegebenenfalls in ein pharmazeutisch verträgliches Salz überführt.

3. Verfahren zur Herstellung von neuen Derivaten gemäß Anspruch 1, dadurch gekennzeichnet, daß man das Reaktionsprodukt des p-Toluol-sulfonylchlorids, des Triethylamins und der Säure der Formel

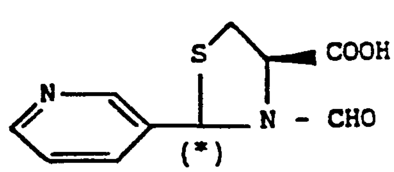

(V)

worin das Symbol * das + Zeichen, das − Zeichen oder das ± Zeichen bedeutet, gemäß dem man ein rechtsdrehendes, linksrehendes oder racemisches Produkt einsetzt, mit dem Reaktionsprodukt von Triethylamin und einem Produkt der allgemeinen Formel

$$ClCH_2—\underset{\underset{Cl}{|}}{CH}—CONHAr \qquad (VIII)$$

worin Ar wie in Anspruch 1 definiert ist, zur Reaktion bringt, dann das erhaltene Produkt isoliert und es gegebenenfalls in ein pharmazeutisch annehmbares Salz überführt.

4. Pharmazeutische Zusammensetzung, dadurch gekennzeichnet, daß sie als aktives Produkt mindestens ein Derivat gemäß Anspruch 1 enthält, zusammen mit einem oder mehreren verträglichen und pharmazeutisch annehmbaren Verdünnungs- oder Hilfsmitteln.

**Patentanspruch für die Vertragsstaaten: AT ES GR**

Verfahren zur Hersellung eines neuen Derivats von 1H,3H-Pyrrolo[1,2-c]thiazol der allgemeinen Formel I:

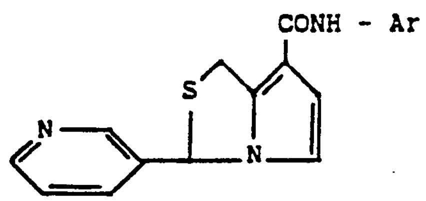

(I)

in welcher Ar einen Pyridylrest, substituiert durch ein oder mehrere Halogenatome oder Alkyl-, Alkyloxy-, Alklthio-, Trifluormethyl-, Amino-, Alkylamino-, Dialkylamino-, Hydroxy-, Cyano-, Alkylsulfinyl-, Alkylsulfonyl-, Sulfamido-, Alkylsulfamido-, Dialkylsulfamido-, Alkylcarbonyl-, Benzoyl-, Alkyloxycarbonyl-, Carboxy-, Phenoxycarbonyl-, Akylcarbonyloxy-, Benzoyloxy-, Alkylcarbonylamino-, Benzamido-, Phenyl-, Benzyl-, Phenoxy- oder Phenylthioreste darstellt, wobei selbstverständlich ist, daß i) die Alkylreste und Alkylteile 1 bis 4 Kohlenstoffatome in gerader oder verzweigter Kette enthalten, ii) die Phenylreste oder Phenylteile gegebenenfalls durch ein oder mehrere Halogenatome oder Alkyl-, Alkyloxy-, Alkylthio-, Trifluormethyl-, Amino-, Alkylamino-, Dialkylamino-, Hydroxy-, Cyano-, Phenyl- oder Benzylreste substituiert sein können und iii) die Erfindung die racemischen Verbindungen, die Enantiomeren und ihre Mischungen sowie die pharmazeutisch zulässigen Salze umfaßt, dadurch gekennzeichnet, daß man:

A — ein Amin der allgemeinen Formel:

$$Ar—NH_2 \qquad (II)$$

in welcher Ar die obige Bedeutung hat, mit einer racemischen oder optisch aktiven Säure der Formel

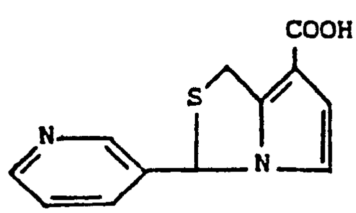

(III)

oder einem aktiven Derivat dieser Säure reagieren läßt, dann das erhaltene Produkt isoliert und es gegebenenfalls in ein pharmazeutisch verträgliches Additionssalz umwandelt oder daß man

B — das Reaktionsprodukt von p-Toluolsulfonylchlorid, Triäthylamin und der Säure der Formel:

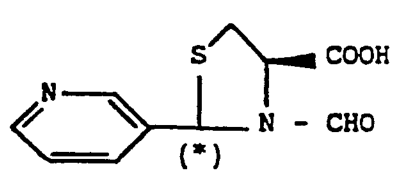

(V)

worin das Symbol * das Zeichen das Zeichen + oder − das Zeichen ± darstellt, je nachdem, ob man eine rechtsdrehende, linksdrehende oder racemisches Verbindung einsetzt, mit dem Reaktionsprodukt von Triethylamin und einer Verbindung der allgemeinen Formel:

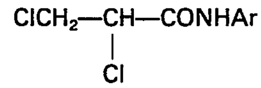

(VIII)

worin Ar die obige Bedeutung hat, reagieren läßt, dann das erhaltene produkt isoliert und es gegebenenfalls in ein pharmazeutisch zulässiges Salz umwandelt.

**Claims for the Contracting States: BE CH DE FR GB IT LI LU NL SE**

1. A new 1H,3H-pyrrolo[1,2-c]thiazole derivative characterized in that it corresponds to the general formula:

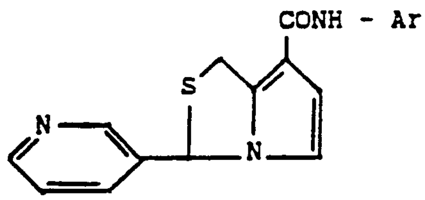

(I)

in which Ar represents a pyridyl radical, substituted by one or more halogen atoms or alkyl, alkyloxy, alkylthio, trifluoromethyl, amino, alkylamino, dialkylamino, hydroxyl, cyano, alkylsulphinyl, alkylsulphonyl, sulphamido, alkyl sulphamido, dialkylsulphamido, alkylcarbonyl, benzoyl, alkyloxycarbonyl, carboxyl, phenoxycarbonyl, alkylcarbonyloxy, benzoyloxy, alkylcarbonylamino, benzamido, phenyl, benzyl, phenoxy or phenylthio radicals, it being understood that i) the alkyl radicals and the alkyl portions contain 1 to 4 carbon atoms in straight- or branched-chain; ii) the phenyl radicals or phenyl portions may be unsubstituted or substituted by one or more halogen atoms or alkyl, alkyloxy, alkylthio, trifluoromethyl, amino, alkylamino, dialkylamino, hydroxyl, cyano, phenyl or benzyl radicals and that iii) the invention relates to the racemic products, the enantiomers and mixtures thereof and to the pharmaceutically acceptable salts.

2. A method for the preparation of new derivatives according to claim 1, characterized in that an amine of general formula:

$$Ar—NH_2 \qquad (II)$$

in which Ar is defined as in Claim 1, is reacted with a racemic or an optically active acid of formula:

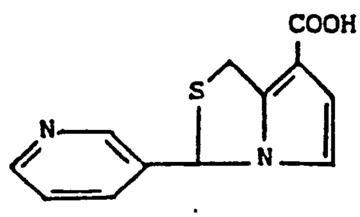

(III)

or a reactive derivative of this acid, and the product obtained is then isolated and converted, if desired, into a pharmaceutically compatible salt.

3. A method for the preparation of new derivatives according to Claim 1, characterized in that the reaction product of p-toluenesulphonyl chloride, triethylamine and the acid of formula:

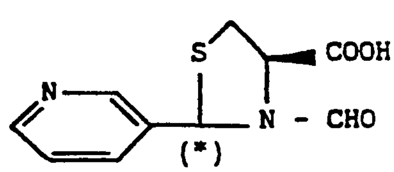

(V)

in which the symbol * represents the sign +, the sign − or the sign ± depending on whether the product employed is dextrorotatory, laevorotatory or racemic, is reacted with the reaction product of triethylamine and a product of general formula:

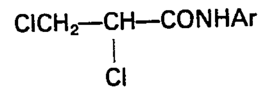

(VIII)

in which Ar is defined as in Claim 1, and the product obtained is then isolated and converted, if desired, into a pharmaceutically acceptable salt.

4. A pharmaceutical composition characterized in that it contains, as active product, at least one derivative according to Claim 1, combined with one or more compatible and pharmaceutically acceptable diluents or adjuvants.

**Claim for the Contracting States: AT ES GR**

A process for the preparation of a new 1H,3H-pyrrolo[1,2-c]thiazole derivative of general formula:

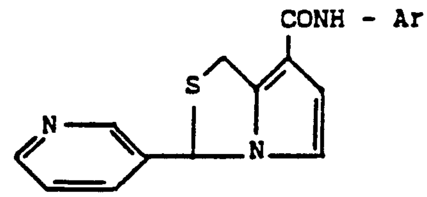

(I)

in which Ar represents a pyridyl radical, substituted by one or more halogen atoms or alkyl, alkyloxy, alkylthio, trifluoromethyl, amino, alkylamino, dialkylamino, hydroxyl, cyano, alkylsulphinyl, alkylsulphonyl, sulphamido, alkylsulphamido, dialkylsulphamido, alkylcarbonyl, benzoyl, alkyloxycarbonyl, carboxyl, phenoxycarbonyl, alkylcarbonyloxy, benzoyloxy, alkylcarbonylamino, benzamido, phenyl, benzyl, phenoxy or phenylthio radicals, it being understood that i) the alkyl radicals and the alkyl portions contain 1 to 4 carbon atoms in straight- or branched-chain; ii) the phenyl radicals or phenyl portions may be unsubstituted or substituted by one or more halogen atoms or alkyl, alkyloxy, alkylthio, trifluoromethyl, amino, alkylamino, dialkylamino, hdyroxyl, cyano, phenyl or benzyl radicals radicals and that iii) the invention relates to the racemic products, the enantiomers and mixtures thereof and to the pharmaceutically acceptable salts, characterized in that:

A — an amine of general formula:

$$Ar—NH_2 \qquad (II)$$

in which Ar is defined as above, is reacted with a racemic or an optically active acid of formula:

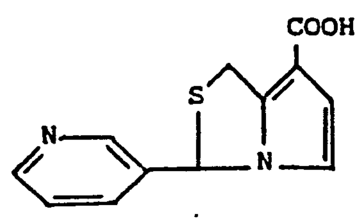

(III)

or a reactive derivative of this acid, and the product obtained is then isolated and converted, if desired, into a pharmaceutically compatible salt or in that

B — the reaction product of p-toluenesulphonyl chloride, of triethylamine and of the acid of formula:

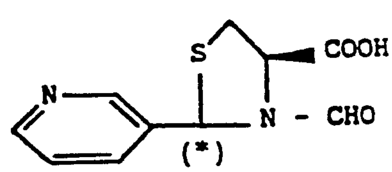

(V)

in which the symbol * represents the sign +, the sign − or the sign ± depending on whether the product employed is dextrorotatory, laevorotatory or racemic, is reacted with the reaction product of triethylamine and of a product of general formula:

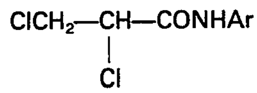

(VIII)

in which Ar is as defined above, and the product obtained is then isolated and converted, if desired, into a pharmaceutically acceptable salt.